# EUROPEAN PATENT APPLICATION

(11) **EP 1 891 945 A1**
(43) Date of publication of application: **27.02.2008**
(21) Application number: 06291239.9
(22) Date of filing: 31.07.2006
(51) Int. Cl.: A61K 31/045, A61K 31/167, A61P 17/02

(54) **Treatment and prevention of excessive scarring**

(71) Applicant: LABORATOIRES BESINS INTERNATIONAL, 75003 Paris (FR); Ascend Therapeutics, Herndon, VA 20170 (US)
(72) Inventor: Masini-Eteve, Valérie, 91370 Verrieres le Buisson (FR); Le Nestour, Elisabeth, 75019 Paris (FR); Bua, Jay, Oakton, Virginia 22124 (US)
(74) Representative: Touati, Catherine

(57) **Abstract**

A pharmaceutical composition comprised of a hydration agent and an anesthetic can be used to treat excessive scarring conditions, such as keloid and hypertrophic scars.

## Description

### Background of the Invention

The present invention relates to the treatment and prevention of excessive scarring, such as is manifested by keloid and hypertrophic scars, with a composition that comprises a hydration agent and an anesthetic.

Keloid scars or "keloids" are overgrowths of dense fibrous tissue that result from variations in normal wound healing. The dense fibrous tissue of a keloid extends beyond the borders of the original wound, and usually does not regress spontaneously. Thus, keloid scarring is out of proportion to the severity of the inciting wound.

Likewise, hypertrophic scars are overgrowths of dense fibrous tissue that result from abnormal wound healing. In contrast to keloids, hypertrophic scars do not extend beyond the original boundaries of a wound. Also unlike keloids, hypertrophic scars typically reach a certain size and then stabilize or regress.

The normal wound healing process extends over a one to two year period, and conceptually consists of three distinct stages. The first stage, the inflammatory stage, is intensely degradative. It begins immediately after injury and provides a means to remove damaged tissues and foreign matter from the wound. A few days after injury, the second stage, the proliferation and matrix synthesis stage, begins. During this stage, fibroblasts from surrounding tissues move into the wound and proliferate. The fibroblasts actively produce collagen, which they secrete into the extracellular matrix. Newly synthesized collagen forms cross-linked fibrils, which provide structural integrity to the wound. After several weeks, the final stage, the remodeling stage, begins. During the remodeling stage, the collagen fibrils, which previously were randomly oriented, align in the direction of mechanical tension, providing further mechanical strength to the wound. Upon completion of the entire process, the skin regains its chemical and physical barrier functions.

Six to eight weeks into the normal wound healing process, anabolic and catabolic processes reach an equilibrium. At this time, scar strength is approximately 30-40% that of healthy skin, and scars typically are hyperemic and thickened. Over the next several months, catabolic and anabolic processes abate, and progressive cross-linking of collagen fibers improves the wound's tensile strength. Also, hyperemia and thickness subside until a flat, white, pliable mature scar develops.

Excessive scarring results from an imbalance in the anabolic and catabolic wound healing processes. In the formation of an excessive scar, more collagen is produced than is degraded. As a result, the scar grows larger than is required for wound healing, with an overproduction of cells, collagen and proteoglycan. Keloids grow in all directions, become elevated above the skin, and remain hyperemic. The exact mechanisms of excessive scarring are poorly understood, but it is believed that common mechanisms underlie the formation of both keloids and hypertrophic scars. Evidence suggests that increased transforming growth factor β1(TGF-β1) expression plays a role in excessive scarring. TGF-β1 promotes extracellular matrix production, and is produced at elevated levels by keloid fibroblasts.

Keloids and hypertrophic scars primarily present a cosmetic concern but can cause contractures, which may result in a loss of function if overlying a joint. Additionally, excessive scars can be painful, pruritic, bear a different pigmentation than surrounding skin and cause a burning sensation. Once keloid lesions occur, they tend to continue growing for weeks to months, even for years. Growth usually progresses slowly, but keloids occasionally enlarge rapidly, even tripling in size within months. As noted above, hypertrophic scars tend to stabilize and regress over time. This regression can be quite slow, however, and often is incomplete.

Management of keloids and hypertrophic scars remains a major unsolved clinical problem. Though many forms of treatment have been used, none has proven to be consistently reliable. Current forms of treatment include use of occlusive dressings, compression therapy, intralesional corticosteroid injections, radiation therapy, and surgery. Experimental therapies for keloids use calcium antagonists, antihistamines, interferons alpha and gamma, topical retinoids, and antiallergic drugs, but whether these experimental therapies are effective is debatable. Studies of them largely are characterized by poor objective response criteria, unknown mechanisms of action and lack of adequate patient follow-up.

Occlusive dressings, such as silicone gel sheets, and pressure devices are unpredictable forms of treatment, as a large percentage of patients treated by these means show little or no improvement. Additionally, compliance with these forms of treatment can be impractical. For example, dressings and pressure devices may need to be worn 24 hours per day for up to 12 months. For a scar on a visible or sensitive location, this simply may not be possible.

Intralesional corticosteroids have been the mainstay of keloid treatment. Corticosteroids reduce excessive scarring by reducing collagen synthesis, altering glucosaminoglycan synthesis, and reducing production of inflammatory mediators and fibroblast proliferation during wound healing. However, roughly half of all keloids fail to respond to corticosteroids, and roughly half of the scars that are completely resolved by corticosteroid treatment recur. Additionally, corticosteroid injections can cause several complications, including atrophy, telangiectasia formation, and skin depigmentation.

Radiation therapy may be the only predictably effective treatment for keloids that is presently available. It has the potential to cause cancer, however, and for this reason it is not generally recommended or accepted as a keloid treatment. Moreover, roughly 20 percent of keloids treated by radiation therapy alone recur within one year.

Surgical procedures, including excision, cryosurgery and laser therapy, can effectively remove keloid tissue, and currently are the treatment of choice for hypertrophic scars. However, these techniques often cause tissue trauma that results in further hypertrophic or keloid scars. Indeed, keloids recur in well more than half of patients treated by surgical excision, cryosurgery, and laser therapy. Additionally, these procedures cause pain and present a risk of infection. Cryosurgery also causes skin depigmentation in some patients.

One proposed therapy for excessive scars entails the administration of selective estrogen receptor modulators, such as tamoxifen and its derivatives. *In vitro,* tamoxifen inhibits keloid fibroblast proliferation and decreases collagen production. Apparently, tamoxifen effects this inhibition by downregulating TGF-β1 expression, which promotes collagen formation. In a related vein, U.S. patent publication No. 2005/0032910 describes treating excessive scarring with 4-hydroxy tamoxifen formulated in a hydroalcoholic gel. Such treatments, however, have not been widely embraced.

Thus, despite the broad array of treatments available, there is no widely accepted and predictably effective means for preventing or treating excessive scars. Accordingly, an effective approach to reducing keloid and hypertrophic scars would offer significant benefit.

### Summary of the Invention

This invention provides a method for treating excessive scarring, illustrated by keloid scars and hypertrophic scars. The method comprises administering an effective amount of a pharmaceutical composition that comprises a hydration agent and an anesthetic for a period of time sufficient to minimize the scar or symptoms of the scar. The invention also provides pharmaceutical compositions for treating excessive scarring. A pharmaceutical composition of the invention does not contain a selective estrogen receptor modulator (SERM), hormone or corticosteroid active ingredient. In certain embodiments, the active ingredients consist solely of a hydration agent and an anesthetic. The treatment approach of the invention offers several advantages over other treatments for scars, including (1) few systemic side effects, (2) a better safety profile, and (3) easy patient compliance.

In performing the inventive method, compositions may be administered by any means that delivers the active ingredients to scar tissue *in vivo.* Preferably, the administration is performed locally, such as by topical administration at the site of a scar or by direct injection into a scar. The inventive methodology may be performed as the sole form of therapy or may be combined with other forms of therapy.

A broad range of topical formulations are suitable for performing the invention. In such formulations, the hydration agent may be an emollient, a gelling agent, a humectant, or a combination thereof. The anesthetic may be any topical anesthetic, such as an alcohol. Preferably, the topical formulation is a hydroalcoholic gel. In a specific example, such gels comprise:
a) about 0.5 % to 2.0 % by weight of isopropyl myristate,
b) about 60% to 75% by weight of absolute alcohol,
c) about 25% to 40% by weight of aqueous vehicle,
d) about 0.5% to 5.0 % by weight of gelling agent,
wherein the percentage of components are weight to weight of the composition.

### Detailed Description of the Preferred Embodiments

The present inventors have discovered that, by administering a pharmaceutical composition comprising a hydration agent and an anesthetic, one can treat excessive scars with few if any side effects. The pharmaceutical compositions do not contain a SERM, hormone or corticosteroid active ingredient. Indeed, in certain embodiments, the only active ingredients in the pharmaceutical compositions are a hydration agent and an anesthetic. Accordingly, the approach of the invention provides a superior safety profile and easier patient compliance compared to other treatment methods.

According to the present invention, the phrases "excessive scar" and "excessive scarring" connote overgrowth of dense fibrous tissue, resulting result from abnormal wound healing. Excessive scars have grown larger than necessary for normal wound healing, and are characterized by overproduction of cells, collagen and/or proteoglycan.

"Keloid scars" are excessive scars in which the dense fibrous tissue extends beyond the borders of the original wound or incision, and does not usually regress spontaneously. Determining whether a scar is a keloid can be a challenge, because keloids often superficially resemble other hypertrophic scars. Still, keloids have distinguishing histological features. One such feature is the collagen nodule, which contains a high density of fibroblasts and unidirectional collagen fibrils in a highly organized and distinct orientation. Additionally, keloids have a rich vasculature, a high mesenchymal cell density, and a thickened epidermal cell layer.

Skin color and genetics, which correlate with keloid formation, also can aid a determination of whether a scar is a keloid. As many as 16% of black Africans have keloids, while Polynesians, Chinese, Indians and Malaysians have fewer. Whites and albinos have the fewest. Patients with keloid scars tend to have an associated strong family history; both autosomal dominant and autosomal recessive modes of transmission have been reported.

"Hypertrophic scars" are excessive scars in which the dense fibrous tissue does not extend beyond the borders of the original wound or incision. They tend to be wider than necessary for normal wound healing to occur. Histologically, hypertrophic scars have more organized collagen fibers than keloids, and scant mucoid matrix. Hypertrophic lesions are characterized by randomly distributed tissue bundles consisting of uniaxially oriented extracellular matrix and cells.

According to the present invention, a "treatment" of an excessive scar refers to a reduction in the defining characteristics or symptoms of the scar. Treatment may be a reduction in the size of a scar, as measured by the length, width, or thickness of the scar. Treatment also may be a reduction in pain associated with the scar or a reduction in itching associated with the scar. Treatment also may be a normalization of pigmentation in the scar, an increase of pliability in the scar or a decrease in vascularity within the scar (which may be evidenced by a color change from purple to red to pink to white within the scar). Improvement in any one of these factors is considered successful treatment.

"Hydration agents" are substances that preserve or increase the moisture content of skin. Studies have shown that water evaporates from excessive scars (e.g., hypertrophic and keloid scars) roughly four times more rapidly than from normal scars and skin. The present inventors have found that stemming that loss of water contributes to treating excessive scars and symptoms of excessive scarring. Hydration agents useful in the invention include emollients, gelling agents and humectants. A combination of hydration agents may be used in the invention.

"Emollients" are substances that soften the skin. Due to their occlusive properties, many emollients improve moisturization of the skin. Emollients are well known in the art, and include mineral oil, petrolatum, polydecene, isohexadecane, fatty acids and alcohols having from 10 to 30 carbon atoms; pelargonic, lauric, myristic, palmitic, stearic, isostearic, hydroxystearic, oleic, linoleic, ricinoleic, arachidic, behenic, and euricic acids and alcohols; triglyceride esters, castor oil, cocoa butter, safflower oil, sunflower oil, jojoba oil, cottonseed oil, corn oil, olive oil, cod liver oil, almond oil, avocado oil, palm oil, sesame oil, squalene, Kikui oil, soybean oil, acetoglyceride esters, ethoxylated glycerides, ethoxylated glyceryl monostearate, alkyl esters of fatty acids having 10 to 20 carbon atoms, hexyl laurate, isohexyl laurate, isohexyl palmitate, isopropyl palmitate, decyl oleate, isodecyl oleate, hexadecyl stearate, decyl stearate, diisopropyl adipate, diisohexyl adipate, diisopropyl sebacate, laurly lactate, myristyl lactate, acetyl lactate; alkenyl esters of fatty acids having 10 to 20 carbon atoms, oleyl myristate, oleyl stearate, oleyl oleate, fatty acid esters of ethoxylated fatty alcohols, polhydric alcohol esters, ethylene glycol mono and di-fatty acid esters, diethylene glycol mono- and di-fatty acid esters, polyethylene glycol, wax esters, beeswax, spermaceti, myristyl myristate, stearyl stearate, silicone oils, dimethicones, cyclomethicones. Emollients that are liquid at room temperature are preferred. A particularly preferred emollient for purposes of the present invention is isopropyl myristate.

"Gelling agents" are substances that increase the viscosity of a liquid formulation. Gelling agents also are well known in the art. Preferred gelling agents include carbomers, cellulose derivatives, poloxamers and poloxamines. More particularly, preferred gelling agents are chitosan, dextran, pectins, natural gum and cellulose derivatives such as ethyl cellulose, hydroxypropyl cellulose, hydroxyethyl cellulose, hydroxypropyl methyl cellulose (HPMC), carboxymethyl cellulose (CMC), and the like. One highly preferred gelling agent is hydroxypropyl cellulose. A combination of gelling agents also can be used in the invention.

"Humectants" are hygroscopic substances that absorb water from the air. Preferred humectants include glycerine, propylene glycol, glyceryl triacetate, a polyol, sorbitol, maltitol, a polymeric polyol, polydextrose, quillaia, lactic acid, urea. Lactic acid and urea are natural components that moisturize, soften and condition the skin. A combination of humectants also can be used in the invention.

"Anesthetics" are substances that reversibly depress neuronal function to produce a loss in the ability to perceive pain and/or other sensations. Local anesthetics have this effect in specific areas of the body or along specific neuronal pathways. For the present invention, topical anesthetics are preferred. These numb the surface of a body part. Preferred topical anesthetics are alcohols, benzocaine, benzyl alcohol, menthol, butamben, dibucaine, lidocaine, pramoxine, and tetracaine. Alcohols are highly preferred, especially lower hydrocarbon chain (C1-C7, preferably C1-C4) alcohols. Among those, ethanol and isopropanol are most highly preferred. In addition to their anesthetic properties, alcohols provide a cooling effect that can reduce burning sensations cause by excessive scars.

For topical administration, a composition used in the invention may be an ointment, cream, gel, emulsion (lotion), powder, oil or similar formulation. To this end, a formulation within the invention may comprise customary excipient additives.

Compositions of the invention preferably are hydroalcoholic gels. The alcohol component used in such gels preferably has a low boiling point, preferably less than 100°C at atmospheric pressure, to permit rapid evaporation upon contact with the scar. Preferred alcoholic components are ethanol and isopropanol. The amount of absolute alcohol in a formulation according to the invention generally ranges between 35% and 99.9%, preferably between 50% and 85%, more preferably between 60% and 75% by weight. Thus, the amount of absolute alcohol in a gel formulation may be about 60%, 61%, 62%, 63%, 64%, 65%, 66%, 67%, 68%, 69%, 70%, 71%, 72%, 73%, 74% or 75% by weight.

The aqueous component used in such gels solubilizes any hydrophilic molecules in a formulation of the invention, and it also promotes moisturization of the skin. An aqueous component also can regulate pH, preferably in the range of about 4 to about 12, more preferably in the range of about 6 to about 11, even more preferably in the range of about 8 to about 10, and most preferably at about 9.

Aqueous components include alkalinizing and basic buffer solutions, including phosphate buffered solutions (e.g., dibasic or monobasic sodium phosphate), citrate buffered solutions (e.g., sodium citrate or potassium citrate) and simply purified water. The phosphate buffer is preferred according to the invention. The amount of an aqueous component preferably ranges between 0.1 % and 65% by weight of the pharmaceutical composition, more preferably between 15% and 50%, and still more preferably between 25% and 40%. Thus, the amount of an aqueous component may be about 25%, 26%, 27%, 28%, 29%, 30%, 31%, 32%, 33%, 34%, 35%, 36%, 37%, 38%, 39% or 40%. In formulations that contain an aqueous component, the amount of absolute alcoholic vehicle in a formulation is preferably from about 60% to about 75%.

The amount of a hydration agent and anesthetic in hydroalcoholic gels of the invention will vary, depending on the particular hydration agent and anesthetic. Determination of pharmaceutically effective amounts for these components is well within the ordinary level of skill. Nevertheless, when isopropyl myristate is used as a hydration agent, the amount in a hydroalcoholic gel may range from about 0.1 to about 5.0 grams per 100 grams of gel. Preferably, the amount of isopropyl myristate ranges from about 0.5 to about 2.0 grams per 100 grams of gel. In such embodiments, isopropyl myristate may constitute about 0.5%, 0.6%, 0.7%, 0.8%, 0.9%, 1.0%, 1.1%, 1.2%, 1.3%, 1.4%, 1.5%, 1.6%, 1.7%, 1.8%, 1.9% or 2.0% by weight of the total composition.

Hydroalcoholic gels may comprise one or more gelling agents to increase the viscosity of a formulation and/or to function as a solubilizing agent. Depending on the gelling agent's nature, it may constitute between 0.1 % and 20% by weight of a formulation, preferably between 0.5% and 10%, more preferably between 0.5% and 5%. Thus, the amount of a gelling agent may be about 0.5%, 1.0%, 1.5%, 2.0%, 2.5%, 3.0%, 3.5%, 4.0%, 4.5% or 5.0%.

When a formulation comprises a gelling agent, in particular a non-preneutralized acrylic polymer, it may advantageously also comprise a neutralizing agent. The neutralizing agent/gelling agent ratio preferably is between 10:1 and 0.1:1, more preferably between 7:1 and 0.5:1, and still more preferably between 4:1 and 1:1. Thus, the neutralizing agent/gelling agent ratio may be about 7:1, 6:1, 5:1, 4:1, 3:1, 2:1, 1:1 or 0.5:1. A neutralizing agent should form, in the presence of the polymer, soluble salts. A neutralizing agent also should permit optimum swelling of polymer chains during neutralization of charges and formation of polymer salts. Useful neutralizing agents include sodium hydroxide, ammonium hydroxide, potassium hydroxide, arginine, aminomethylpropanol, trolamine and tromethamine. Those skilled in the art will select a neutralizing agent according to the type of gelling agent employed in a formulation. When cellulose derivatives are used as gelling agents, however, no neutralizing agents are required.

In certain embodiments, the hydroalcoholic gel comprises:
a) about 0.5% to 2% by weight of isopropyl myristate,
b) about 60% to 75% by weight of absolute alcohol,
c) about 25% to 40% by weight of aqueous component, and
d) about 0.5% to 5% by weight of gelling agent,
wherein the percentage of components are weight to weight of said composition. Thus, isopropyl myristate may constitute about 0.5%, 0.6%, 0.7%, 0.8%, 0.9%, 1.0%, 1.1%, 1.2%, 1.3%, 1.4%, 1.5%, 1.6%, 1.7%, 1.8%, 1.9% or 2.0% by weight of the hydroalcoholic gel. In highly preferred embodiments, ethyl alcohol or isopropanol is the alcoholic component; polyacrylic acid, hydroxypropyl cellulose or another cellulose derivative is the gelling agent; and a phosphate buffered solution is the aqueous component.

As noted above, compositions of the invention do not contain a SERM, hormone or corticosteroid active ingredient. SERMs constitute a class of compounds that act on estrogen receptors and exert various effects, depending on their metabolism and the tissue. That is, SERMs selectively inhibit or stimulate estrogen-like action in the tissues. Members of the SERM class include tamoxifen, 4-hydroxy tamoxifen, raloxifene, clomifene, toremifene, bazedoxifene, lasofoxifene, tibolone, droloxifene, idoxifene, ormeloxifene, arzoxifen, ospemifene, nafoxidene, trioxifene, and antibodies that bind to estrogen or estrogen receptors.

Hormones, as used herein, refers to steroid hormones. The steroid hormones are fat-soluble organic compounds based on a structure of 17 carbon atoms arranged in four rings. They can be grouped into five groups based on the receptors to which they bind: glucocorticoids, mineralocorticoids, androgens, estrogens and progestagens.

Glucocorticoids, or corticosteroids, bind with the cortisol receptor. Cortisol, also known as hydrocortisone, is the prototypical glucocorticoid. Other glucocorticoids are cortisone acetate, prednisone, prednisolone, methylprednisolone, dexamethasone, betamethasone, triamcinolone, beclometasone, fludrocortisone acetate, deoxycorticosterone acetate (DOCA) and aldosterone.

Mineralocorticoids bind to the cytosolic mineralocorticoid receptor. Aldosterone is a primary example. An exemplary synthetic mineralocorticoid is fludrocortisone.

Androgens bind to androgen receptors. The primary androgen is testosterone. Others are dehydroepiandrosterone (DHEA), dehydropiandrosterone sulfate (DHEA-S), androstenedione, androstanediol, androsterone and dihydrotestosterone (DHT). A number of synthetic androgens exist, including oxandrolone and decadurabolin.

Estrogens bind to estrogen receptors. Primary examples include estradiol, estriol and estrone. Diethylstilbestrol is a synthetic example.

Progestagens bind to the progesterone receptor. Progesterone in the primary example. Others include cyproterone acetate, dydrogesterone, medroxyprogesterone acetate, chlormadinone acetate, megestrol, promegstone, norethisterone, norethindrone acetate, (levo)norgestrel, lynestrenol, desogestrel, drospirenone, ehtynodiol diacetate, norelgestromin, norgestimate, gestodene and tibolone.

According to the present invention, a pharmaceutical composition may be administered in any dosage form and via any system that delivers the active ingredients to a scar *in vivo.* Preferably, the composition is topically applied to the surface of a scar or is injected into scar. The optimal method of administration to treat scarring will depend upon the location of the scar and the extent of scarring.

Determination of a dosage and administration schedule are within the ordinary skill of the art. In general, appropriate dosages and administration schedules can be defined by routine testing in order to obtain optimal treatment of scars, while minimizing any potential side effects. Moreover, the dosage and administration schedules may be optimized using a pharmacokinetic/pharmacodynamic modeling system. For example, one or more dosage regimens may be chosen and a pharmacokinetic/pharmacodynamic model may be used to determine the pharmacokinetic/pharmacodynamic profile of one or more dosage regimens. Next, one of the dosage regimens for administration may be selected which achieves the desired pharmacokinetic/pharmacodynamic response based on the particular pharmacokinetic/pharmacodynamic profile.

Specifically, pharmaceutical compositions may be administered at least once a week and preferably at least once a day over the course of several weeks to several months. In one embodiment, the compositions are administered at least once a week or at least once a day over one to six months. Prolonged use of the compositions is possible due to the excellent safety profile.

More specifically, the pharmaceutical compositions may be administered at least once a day for about 2 weeks, at least once a day for about 3 weeks, at least once a day for about 4 weeks, at least once a day for about 5 weeks, at least once a day for about 6 weeks, at least once a day for about 7 weeks, at least once a day for about 2 months, at least once a day for about 3 months, at least once a day for about 4 months, at least once a day for about 5 months, at least once a day for about 6 months, at least once a day for about 7 months, at least once a day for about 8 months, at least once a day for about 9 months, at least once a day for about 10 months, at least once a day for about 11 months, or at least once a day for about a year or more.

Alternatively, the pharmaceutical compositions may be administered about once every day, about twice every day, about thrice every day, about once every 2 days, about once every 3 days, about once every 4 days, about once every 5 days, about once every 6 days or about once every 7 days for the periods set forth above.

It is contemplated that the present invention may be combined with other keloid therapies. According to the present invention, therefore, the described method of treatment may be accompanied by the use of occlusive dressings, compression therapy, intralesional corticosteroid injections, radiation therapy, and surgery, including cryotherapy and laser therapy.

Reference to the following illustrative example will help to provide a more complete understanding of the invention.

### Example 1: Keloid clinical experience with an hydro alcoholic gel.

This example demonstrates that a hydroalcoholic gel containing a hydration agent and an anaesthetic, without 4-hydroxy tamoxifen or other active agent, reduces the symptoms of keloid scars.

A six-month study was performed on 62 women having keloid scars. Selection criteria for the study required participants to be over 18 years of age and to present with a symptomatic keloid scar as assessed by a minimum 40 mm level on the Visual Analog Scale for pain/discomfort and/or itching. None of the scars had been treated with any other agent, including steroids, external radiation or silicone sheeting within the previous three months. As noted earlier, keloid scars generally do not regress spontaneously.

The participants were randomized into two groups. In a first group, 32 women applied 0.5 ml of hydroalcoholic gel to their keloid scar twice daily for six months. The hydroalcoholic gel contained 1% isopropyl myristate, 1.5% hydroxypropyl cellulose, 70.9% ethanol (96% alcohol v/v), and 26.5% phosphate buffer solution. In the second group, 30 women applied 0.5 ml of a nearly identical hydroalcoholic gel, containing a selective estrogen receptor modulator (SERM), to their keloid scar twice daily for six months. The only difference between the two gel compositions was the presence or absence of 0.05% SERM. Both gels were transparent, odourless, and dried within two minutes of being spread on the scars. They were dispensed utilizing a canister with a metered airless pump.

The study was conducted at a specialized hospital site by dermatologists. Follow-up visits were conducted with the participants at months 1, 3 and 6. Compliance to gel application throughout the study was 98% for both groups.

Results of the study were as follows:
(a) The response rate, defined as having over 10% decrease in pain/discomfort was 65% at the end of Month 1, reached 68% at Month 2-5, and 66% at Month 6.
(b) The response rate, defined as having over 10% decrease in itching was 59% at the end of Month 1, reached 72% at Month 2, 78 % at Month 3 and 4, 62% at Month 5, and 69% at Month 6.
(c) The overall change in pain/discomfort (measured by visual analog scales (VAS)) was a decrease from a baseline mean of 59±25 to 37±28 at Month 1, and 32±32 at Month 6.
(d) The overall change in itching on the VAS was a decrease from a baseline mean of 69±22 to 45±31 at Month 1, and 35±31 at Month 6.
These results were confirmed by the clinical global impressions of the clinician and the subject :
(a) The percent of the study population that felt much or very much improved was 29% at Month 3 and 39% at Month 6.
(b) The clinician assessed as much or very much improved 32% of the patients at Month 3 and 35% at Month 6.
(c) The safety and tolerance of the topical gel application was excellent.

Efficacy data were generated for two analysis populations. The primary analysis population was an Intent-to-Treat (ITT) population, which included all subjects randomized to the two groups described above who had at least one treatment efficacy measurement. A secondary analysis population was a Per-Protocol population, which included all subjects who adhered to the protocol by (a) participating in the study through at least Month 4, (b) having at least one valid efficacy assessment after Month 4, (c) having no serious violation of protocol requirements, and (d) complying with application of the hydroalcoholic gel. Analysis of the data showed the following results:
(a) Both the ITT and Per-Protocol populations experienced statistically significant improvements in pain/discomfort (p < 0.001) over baseline at Months 1, 2, 3, 4, 5 and 6.
(b) Both the ITT and Per-Protocol populations experienced statistically significant improvements in itching (p < 0.001) over baseline at Months 1, 2, 3, 4, 5 and 6..
(c) Both the ITT and Per-Protocol populations experienced statistically significant improvements in pigmentation, pliability and height over baseline at Months 3 and 6. Both populations also experiences statistically significant improvements in vascularity at Month 6.
The following tables provide details of the statistical analyses.

Table 1.1 shows that application of the hydroalcoholic gel effected a statistically significant improvement in pain/discomfort within the ITT population.

**Table 1.1**

| Pain/Discomfort VAS Score ITT Population | | | | | | | |
|---|---|---|---|---|---|---|---|
| | | Hydroalcoholic Gel | | | | | |
| | | N=32 | | | | | |
| Visit | | N | Mean | Median | SD | Range | P-value# |
| VAS Score* (mm) | | | | | | | |
| Baseline | | | | | | | |
| | Value | 32 | 59.0 | 56.5 | 25.6 | (1.0,100.0) | |
| Month 1 | | | | | | | |
| | Value | 32 | 37.0 | 39.0 | 28.2 | (0.0, 99.0) | |
| | Change** | 32 | -21.9 | -20.5 | 32.7 | (-95.0, 64.0) | <0.001 |
| Month 2 | | | | | | | |
| | Value | 32 | 36.5 | 34.5 | 29.3 | (0.0,100.0) | |
| | Change** | 32 | -22.4 | -26.0 | 27.7 | (-91.0, 24.0) | <0.001 |
| Month 3 | | | | | | | |
| | Value | 32 | 31.7 | 26.5 | 30.9 | (0.0,100.0) | |
| | Change** | 32 | -27.3 | -27.0 | 32.6 | (-100, 38.0) | <0.001 |
| Month 4 | | | | | | | |
| | Value | 32 | 33.9 | 26.0 | 28.1 | (0.0,100.0) | |
| | Change** | 32 | -25.1 | -17.0 | 30.1 | (-91.0, 22.0) | <0.001 |
| Month 5 | | | | | | | |
| | Value | 32 | 33.3 | 26.5 | 30.9 | (0.0,100.0) | |
| | Change** | 32 | -25.7 | -15.5 | 32.6 | (-100, 20.0) | <0.001 |
| Month 6 | | | | | | | |
| | Value | 32 | 32.0 | 19.0 | 31.6 | (0.0, 99.0) | |
| | Change** | 32 | -27.0 | -25.0 | 32.9 | (-91.0, 46.0) | <0.001 |

| | | | | | | | |
|---|---|---|---|---|---|---|---|
| * The rating was obtained using 100 mm VAS, with 0='No pain/discomfort' and 100='Worst pain/discomfort imaginable.' ** Change = Change from baseline. # P-value is from a paired t-test. | | | | | | | |

Table 1.2 shows that application of the hydroalcoholic gel effected a statistically significant improvement in pain/discomfort within the per protocol population.

**Table 1.2**

| Pain/Discomfort VAS Score Per Protocol Population | | | | | | | |
|---|---|---|---|---|---|---|---|
| | | Hydroalcoholic Gel | | | | | |
| | | N=31 | | | | | |
| Visit | | N | Mean | Median | SD | Range | P-value# |
| VAS Score* (mm) | | | | | | | |
| Baseline | | | | | | | |
| | Value | 31 | 58.3 | 56.0 | 25.8 | (1.0,100.0) | |
| Month 1 | | | | | | | |
| | Value | 31 | 35.5 | 38.0 | 27.4 | (0.0, 99.0) | |
| | Change** | 31 | -22.8 | -22.0 | 32.9 | (-95.0, 64.0) | <0.001 |
| Month 2 | | | | | | | |
| | Value | 31 | 35.3 | 34.0 | 28.9 | (0.0,100.0) | |
| | Change** | 31 | -23.0 | -26.0 | 28.0 | (-91.0, 24.0) | <0.001 |
| Month 3 | | | | | | | |
| | Value | 31 | 30.3 | 26.0 | 30.3 | (0.0,100.0) | |
| | Change** | 31 | -28.1 | -28.0 | 32.8 | (-100, 38.0) | <0.001 |
| Month 4 | | | | | | | |
| | Value | 31 | 32.5 | 26.0 | 27.6 | (0.0,100.0) | |
| | Change** | 31 | -25.8 | -18.0 | 30.3 | (-91.0, 22.0) | <0.001 |
| Month 5 | | | | | | | |
| | Value | 31 | 32.0 | 26.0 | 30.4 | (0.0,100.0) | |
| | Change** | 31 | -26.4 | -17.0 | 32.9 | (-100, 20.0) | <0.001 |
| Month 6 | | | | | | | |
| | Value | 31 | 30.6 | 16.0 | 31.1 | (0.0, 99.0) | |
| | Change** | 31 | -27.7 | -25.0 | 33.2 | (-91.0, 46.0) | <0.001 |

| | | | | | | | |
|---|---|---|---|---|---|---|---|
| * The rating was obtained using 100 mm VAS, with 0='No pain/discomfort' and 100='Worst pain/discomfort imaginable.' ** Change = Change from baseline. # P-value is from a paired t-test. | | | | | | | |

Table 2.1 shows that application of the hydroalcoholic gel effected a statistically significant improvement in itching within the ITT population.

**Table 2.1**

| Itching VAS Score ITT Population | | | | | | | |
|---|---|---|---|---|---|---|---|
| | | Hydroalcoholic Gel | | | | | |
| | | N=32 | | | | | |
| Visit | | N | Mean | Median | SD | Range | P-value# |
| VAS Score* (mm) | | | | | | | |
| Baseline | | | | | | | |
| | Value | 32 | 68.9 | 75.5 | 21.7 | (1.0,100.0) | |
| Month 1 | | | | | | | |
| | Value | 32 | 44.8 | 47.5 | 31.1 | (0.0, 95.0) | |
| | Change** | 32 | -24.1 | -19.5 | 31.7 | (-94.0, 21.0) | <0.001 |
| Month 2 | | | | | | | |
| | Value | 32 | 42.8 | 48.5 | 27.1 | (1.0, 93.0) | |
| | Change** | 32 | -26.2 | -19.5 | 29.1 | (-95.0, 19.0) | <0.001 |
| Month 3 | | | | | | | |
| | Value | 32 | 40.0 | 40.5 | 28.7 | (0.0, 94.0) | |
| | Change** | 32 | -28.9 | -29.5 | 36.1 | (-99.0, 88.0) | <0.001 |
| Month 4 | | | | | | | |
| | Value | 32 | 35.8 | 36.5 | 28.7 | (0.0, 92.0) | |
| | Change** | 32 | -33.1 | -32.0 | 30.6 | (-100, 16.0) | <0.001 |
| Month 5 | | | | | | | |
| | Value | 32 | 39.1 | 37.5 | 30.9 | (0.0, 91.0) | |
| | Change** | 32 | -29.8 | -30.5 | 34.4 | (-100, 17.0) | <0.001 |
| Month 6 | | | | | | | |
| | Value | 32 | 35.3 | 29.3 | 31.2 | (0.0, 92.0) | |
| | Change** | 32 | -33.6 | -35.0 | 34.6 | (-100, 32.0) | <0.001 |

| | | | | | | | |
|---|---|---|---|---|---|---|---|
| * The rating was obtained using 100 mm VAS, with 0='No itching' and 100='Worst itching imaginable.' ** Change = Change from baseline. # P-value is from a paired t-test. | | | | | | | |

Table 2.2 shows that application of the hydroalcoholic gel effected a statistically significant improvement in itching within the per protocol population.

**Table 2.2**

| Itching VAS Score Per Protocol Population | | | | | | | |
|---|---|---|---|---|---|---|---|
| | | Hydroalcoholic Gel | | | | | |
| | | N=31 | | | | | |
| Visit | | N | Mean | Median | SD | Range | P-value# |
| VAS Score* (mm) | | | | | | | |
| Baseline | | | | | | | |
| | Value | 31 | 68.7 | 75.0 | 22.1 | (1.0,100.0) | |
| Month 1 | | | | | | | |
| | Value | 31 | 43.4 | 45.0 | 30.6 | (0.0, 95.0) | |
| | Change** | 31 | -25.3 | -20.0 | 31.5 | (-94.0, 21.0) | <0.001 |
| Month 2 | | | | | | | |
| | Value | 31 | 41.7 | 48.0 | 26.9 | (1.0, 93.0) | |
| | Change** | 31 | -27.0 | -20.0 | 29.2 | (-95.0, 19.0) | <0.001 |
| Month 3 | | | | | | | |
| | Value | 31 | 38.9 | 40.0 | 28.4 | (0.0, 94.0) | |
| | Change** | 31 | -29.8 | -30.0 | 36.3 | (-99.0, 88.0) | <0.001 |
| Month 4 | | | | | | | |
| | Value | 31 | 34.6 | 36.0 | 28.2 | (0.0, 92.0) | |
| | Change** | 31 | -34.1 | -33.0 | 30.5 | (-100, 16.0) | <0.001 |
| Month 5 | | | | | | | |
| | Value | 31 | 38.0 | 36.0 | 30.7 | (0.0, 91.0) | |
| | Change** | 31 | -30.7 | -31.0 | 34.5 | (-100, 17.0) | <0.001 |
| Month 6 | | | | | | | |
| | Value | 31 | 34.1 | 27.5 | 30.8 | (0.0, 92.0) | |
| | Change** | 31 | -34.6 | -39.0 | 34.6 | (-100, 32.0) | <0.001 |

| | | | | | | | |
|---|---|---|---|---|---|---|---|
| * The rating was obtained using 100 mm VAS, with 0='No itching' and 100='Worst itching imaginable.' ** Change = Change from baseline. # P-value is from a paired t-test. | | | | | | | |

Table 3.1 shows that application of the hydroalcoholic gel effected statistically significant improvements in pigmentation, pliability, height, vascularity and total Vancouver Scar Scale Rating score within the ITT population.

**Table 3.1**

| Vancouver Scar Scale Rating ITT Population | | | |
|---|---|---|---|
| | | | Hydroalcoholic Gel |
| Pigmentation | Baseline | 0 - Normal | 1(3.1) |
| | | 1 - Hypopigmented | 2(6.3) |
| | | 2 - Mixed | 17(53.1) |
| | | 3 - Hyperpigmented | 12(37.5) |
| | | | |
| | | Score | |
| | | N | 32 |
| | | Mean | 2.3 |
| | | Median | 2.0 |
| | | SD | 0.7 |
| | | Range | (0,3) |
| | | | |
| | Month 3 | 0 - Normal | 5(17.9) |
| | | 1 - Hypopigmented | 1(3.6) |
| | | 2 - Mixed | 17(60.7) |
| | | 3 - Hyperpigmented | 5(17.9) |
| | | | |
| | | Score | |
| | | N | 28 |
| | | Mean | 1.8 |
| | | Median | 2.0 |
| | | SD | 1.0 |
| | | Range | (0,3) |
| | | P-value* | 0.013 |
| | Month 6 | 0 - Normal | 7(22.6) |
| | | 1 - Hypopigmented | 1(3.2) |
| | | 2 - Mixed | 15(48.4) |
| | | 3 - Hyperpigmented | 8(25.8) |
| | | | |
| | | Score | |
| | | N | 31 |
| | | Mean | 1.8 |
| | | Median | 2.0 |
| | | SD | 1.1 |
| | | Range | (0,3) |
| | | P-value* | 0.033 |
| | | | |
| Pliability | Baseline | 0 - Normal | 0(0.0) |
| | | 1 - Supple | 2(6.3) |
| | | 2 - Yielding | 9(28.1) |
| | | 3 - Firm | 15 (46. 9) |
| | | 4 - Ropes | 5(15.6) |
| | | 5 - Contracture | 1(3.1) |
| | | | |
| | | Score | |
| | | N | 32 |
| | | Mean | 2.8 |
| | | Median | 3.0 |
| | | SD | 0.9 |
| | | Range | (1,5) |
| | | | |
| | Month 3 | 0 - Normal | 0(0.0) |
| | | 1 - Supple | 12(42.9) |
| | | 2 - Yielding | 11(39.3) |
| | | 3 - Firm | 5(17.9) |
| | | 4 - Ropes | 0(0.0) |
| | | 5 - Contracture | 0(0.0) |
| | | | |
| | | Score | |
| | | N | 28 |
| | | Mean | 1.8 |
| | | Median | 2.0 |
| | | SD | 0.8 |
| | | Range | (1,3) |
| | | P-value* | <0.001 |
| | | | |
| | Month 6 | 0 - Normal | 2(6.5) |
| | | 1 - Supple | 12(38.7) |
| | | 2 - Yielding | 9(29.0) |
| | | 3 - Firm | 3(9.7) |
| | | 4 - Ropes | 4(12.9) |
| | | 5 - Contracture | 1(3.2) |
| | | | |
| | | Score | |
| | | N | 31 |
| | | Mean | 1.9 |
| | | Median | 2.0 |
| | | SD | 1.3 |
| | | Range | (0,5) |
| | | P-value* | <0.001 |
| | | | |
| Height | Baseline | 0 - Flat | 0(0.0) |
| | | 1 - <2mm | 6(18.8) |
| | | 2 - 2-5mm | 20(62.5) |
| | | 3 - >5mm | 6(18.8) |
| | | | |
| | | Score | |
| | | N | 32 |
| | | Mean | 2.0 |
| | | Median | 2.0 |
| | | SD | 0.6 |
| | | Range | (1,3) |
| | | | |
| | Month 3 | 0 - Flat | 1(3.6) |
| | | 1 - <2mm | 10 (35.7) |
| | | 2 - 2-5mm | 11(39.3) |
| | | 3 - >5mm | 6(21.4) |
| | | | |
| | | Score | |
| | | N | 28 |
| | | Mean | 1.8 |
| | | Median | 2.0 |
| | | SD | 0.8 |
| | | Range | (0,3) |
| | | P-value* | 0.043 |
| | | | |
| | Month 6 | 0 - Flat | 0(0.0) |
| | | 1 - <2mm | 12 (38.7) |
| | | 2 - 2-5mm | 14(45.2) |
| | | 3 - >5mm | 5 (16.1) |
| | | | |
| | | Score | |
| | | N | 31 |
| | | Mean | 1.8 |
| | | Median | 2.0 |
| | | SD | 0.7 |
| | | Range | (1,3) |
| | | P-value* | 0.032 |
| | | | |
| Vascularity | Baseline | 0 - Normal | 16(50.0) |
| | | 1 - Pink | 13(40.6) |
| | | 2 - Red | 3(9.4) |
| | | 3 - Purple | 0(0.0) |
| | | | |
| | | Score | |
| | | N | 32 |
| | | Mean | 0.6 |
| | | Median | 0.5 |
| | | SD | 0.7 |
| | | Range | (0,2) |
| | | 0 - Normal | 19(67.9) |
| | | 1 - Pink | 8(28.6) |
| | | 2 - Red | 1(3.6) |
| | | 3 - Purple | 0(0.0) |
| | | | |
| | | Score | |
| | | N | 28 |
| | | Mean | 0.4 |
| | | Median | 0.0 |
| | | SD | 0.6 |
| | | Range | (0,2) |
| | | P-value* | 0.11 |
| | | | |
| | Month 6 | 0 - Normal | 21(67.7) |
| | | 1 - Pink | 10(32.3) |
| | | 2 - Red | 0(0.0) |
| | | 3 - Purple | 0(0.0) |
| | | | |
| | | Score | |
| | | N | 31 |
| | | Mean | 0.3 |
| | | Median | 0.0 |
| | | SD | 0.5 |
| | | Range | (0,1) |
| | | P-value* | 0.009 |
| | | | |
| Total score | Baseline | N | 32 |
| | | Mean | 7.7 |
| | | Median | 8.0 |
| | | SD | 1.4 |
| | | Range | (4,9) |
| | | | |
| | Month 3 | N | 28 |
| | | Mean | 5.7 |
| | | Median | 5.0 |
| | | SD | 1.7 |
| | | Range | (2,9) |
| | | P-value* | <0.001 |
| | | | |
| | Month 6 | N | 31 |
| | | Mean | 5.8 |
| | | Median | 6.0 |
| | | SD | 2.2 |
| | | Range | (1,9) |
| | | P-value* | <0.001 |

| | | | |
|---|---|---|---|
| *P-value is from a paired t-test comparing the post-baseline score to the baseline score. | | | |

Table 3.2 shows that application of the hydroalcoholic gel effected statistically significant improvements in pigmentation, pliability, height, vascularity and total Vancouver Scar Scale Rating score within the per protocol population.

**Table 3.2**

| Vancouver Scar Scale Rating Per Protocol Population | | | |
|---|---|---|---|
| | | | Hydroalcoholic Gel |
| Pigmentation | Baseline | 0 - Normal | 1(3.2) |
| | | 1 - Hypopigmented | 2(6.5) |
| | | 2 - Mixed | 16(51.6) |
| | | 3 - Hyperpigmented Score | 12(38.7) |
| | | | |
| | | N | 31 |
| | | Mean | 2.3 |
| | | Median | 2.0 |
| | | SD | 0.7 |
| | | Range | (0,3) |
| | | | |
| | Month 3 | 0 - Normal | 5(17.9) |
| | | 1 - Hypopigmented | 1(3.6) |
| | | 2 - Mixed | 17(60.7) |
| | | 3 - Hyperpigmented Score | 5(17.9) |
| | | | |
| | | N | 28 |
| | | Mean | 1.8 |
| | | Median | 2.0 |
| | | SD | 1.0 |
| | | Range | (0,3) |
| | | P-value* | 0.013 |
| | | | |
| | Month 6 | 0 - Normal | 7(22.6) |
| | | 1 - Hypopigmented | 1(3.2) |
| | | 2 - Mixed | 15(48.4) |
| | | 3 - Hyperpigmented | 8(25.8) |
| | | | |
| | | Score | |
| | | N | 31 |
| | | Mean | 1.8 |
| | | Median | 2.0 |
| | | SD | 1.1 |
| | | Range | (0,3) |
| | | P-value* | 0.033 |
| | | | |
| Pliability | Baseline | 0 - Normal | 0(0.0) |
| | | 1 - Supple | 2(6.5) |
| | | 2 - Yielding | 9(29.0) |
| | | 3 - Firm | 14(45.2) |
| | | 4 - Ropes | 5(16.1) |
| | | 5 - Contracture | 1(3.2) |
| | | | |
| | | Score | |
| | | N | 31 |
| | | Mean | 2.8 |
| | | Median | 3.0 |
| | | SD | 0.9 |
| | | Range | (1,5) |
| | | | |
| | Month 3 | 0 - Normal | 0(0.0) |
| | | 1 - Supple | 12(42.9) |
| | | 2 - Yielding | 11(39.3) |
| | | 3 - Firm | 5(17.9) |
| | | 4 - Ropes | 0(0.0) |
| | | 5 - Contracture | 0(0.0) |
| | | | |
| | | Score | |
| | | N | 28 |
| | | Mean | 1.8 |
| | | Median | 2.0 |
| | | SD | 0.8 |
| | | Range | (1,3) |
| | | P-value* | <0.001 |
| | | | |
| | Month 6 | 0 - Normal | 2(6.5) |
| | | 1 - Supple | 12(38.7) |
| | | 2 - Yielding | 9(29.0) |
| | | 3 - Firm | 3(9.7) |
| | | 4 - Ropes | 4(12.9) |
| | | 5 - Contracture | 1(3.2) |
| | | | |
| | | Score | |
| | | N | 31 |
| | | Mean | 1.9 |
| | | Median | 2.0 |
| | | SD | 1.3 |
| | | Range | (0,5) |
| | | P-value* | <0.001 |
| | | | |
| Height | Baseline | 0 - Flat | 0(0.0) |
| | | 1 - <2mm | 6(19.4) |
| | | 2 - 2-5mm | 19(61.3) |
| | | 3 - >5mm | 6(19.4) |
| | | | |
| | | Score | |
| | | N | 31 |
| | | Mean | 2.0 |
| | | Median | 2.0 |
| | | SD | 0.6 |
| | | Range | (1,3) |
| | | | |
| | Month 3 | 0 - Flat | 1(3.6) |
| | | 1 - <2mm | 10 (35.7) |
| | | 2 - 2-5mm | 11(39.3) |
| | | 3 - >5mm | 6(21.4) |
| | | | |
| | | Score | |
| | | N | 28 |
| | | Mean | 1.8 |
| | | Median | 2.0 |
| | | SD | 0.8 |
| | | Range | (0,3) |
| | | P-value* | 0.043 |
| | | | |
| | Month 6 | 0 - Flat | 0(0.0) |
| | | 1 - <2mm | 12 (38.7) |
| | | 2 - 2-5mm | 14(45.2) |
| | | 3 - >5mm | 5(16.1) |
| | | | |
| | | Score | |
| | | N | 31 |
| | | Mean | 1.8 |
| | | Median | 2.0 |
| | | SD | 0.7 |
| | | Range | (1,3) |
| | | P-value* | 0.032 |
| | | | |
| Vascularity | Baseline | 0 - Normal | 16(51.6) |
| | | 1 - Pink | 12(38.7) |
| | | 2 - Red | 3(9.7) |
| | | 3 - Purple | 0(0.0) |
| | | | |
| | | Score | |
| | | N | 31 |
| | | Mean | 0.6 |
| | | Median | 0.0 |
| | | SD | 0.7 |
| | | Range | (0,2) |
| | | | |
| | Month 3 | 0 - Normal | 19(67.9) |
| | | 1 - Pink | 8 (28. 6) |
| | | 2 - Red | 1(3.6) |
| | | 3 - Purple | 0(0.0) |
| | | | |
| | | Score | |
| | | N | 28 |
| | | Mean | 0.4 |
| | | Median | 0.0 |
| | | SD | 0.6 |
| | | Range | (0,2) |
| | | P-value* | 0.11 |
| | | | |
| | Month 6 | 0 - Normal | 21(67.7) |
| | | 1 - Pink | 10(32.3) |
| | | 2 - Red | 0(0.0) |
| | | 3 - Purple | 0(0.0) |
| | | | |
| | | Score | |
| | | N | 31 |
| | | Mean | 0.3 |
| | | Median | 0.0 |
| | | SD | 0.5 |
| | | Range | (0,1) |
| | | P-value* | 0.009 |
| | | | |
| Total score | Baseline | N | 31 |
| | | Mean | 7.6 |
| | | Median | 8.0 |
| | | SD | 1.4 |
| | | Range | (4,9) |
| | | | |
| | Month 3 | N | 28 |
| | | Mean | 5.7 |
| | | Median | 5.0 |
| | | SD | 1.7 |
| | | Range | (2,9) |
| | | P-value* | <0.001 |
| | | | |
| | Month 6 | N | 31 |
| | | Mean | 5.8 |
| | | Median | 6.0 |
| | | SD | 2.2 |
| | | Range | (1,9) |
| | | P-value* | <0.001 |

| | | | |
|---|---|---|---|
| * P-value is from a paired t-test comparing the post-baseline score to the baseline score. | | | |

Table 4.1 shows that application of the hydroalcoholic gel effected a statistically significant improvement in global impression of change in keloid scar severity within the ITT population.

**Table 4.1**

| Clinical and Subject's Global Impression of Change in Keloid Scar Severity ITT Population | | | | | | |
|---|---|---|---|---|---|---|
| | Hydroalcoholic Gel | | | | | |
| | N | Mean | Median | SD | Range | P-value* |
| Clinical Global Impression of Change | | | | | | |
| Month 3 | 28 | 3.0 | 3.0 | 1.0 | (1.0-5.0) | <0.001 |
| Month 6 | 31 | 2.8 | 3.0 | 1.0 | (1.0-5.0) | <0.001 |
| | | | | | | |
| Subject's Global Impression of Change | | | | | | |
| Month 3 | 28 | 3.0 | 3.0 | 1.1 | (1.0-5.0) | <0.001 |
| Month 6 | 31 | 2.9 | 3.0 | 1.0 | (1.0-5.0) | <0.001 |

| | | | | | | |
|---|---|---|---|---|---|---|
| CGIC and SGIC score: 1=Very Much Improved, 2=Much Improved, 3=Minimally Improved, 4=No Change, 5= Minimally Worse, 6=Much Worse, 7=Very Much Worse. * P-value is from a t-test comparing the mean score to a score of 4. | | | | | | |

Table 4.2 shows that application of the hydroalcoholic gel effected a statistically significant improvement in global impression of change in keloid scar severity within the protocol population.

**Table 4.2**

| Clinical and Subject's Global Impression of Change in Keloid Scar Severity Per Protocol Population | | | | | | |
|---|---|---|---|---|---|---|
| | Hydroalcoholic Gel | | | | | |
| | N | Mean | Median | SD | Range | P-value* |
| Clinical Global Impression of Change | | | | | | |
| Month 3 | 28 | 3.0 | 3.0 | 1.0 | (1.0-5.0) | <0.001 |
| Month 6 | 31 | 2.8 | 3.0 | 1.0 | (1.0-5.0) | <0.001 |
| | | | | | | |
| Subject's Global Impression of Change | | | | | | |
| Month 3 | 28 | 3.0 | 3.0 | 1.1 | (1.0-5.0) | <0.001 |
| Month 6 | 31 | 2.9 | 3.0 | 1.0 | (1.0-5.0) | <0.001 |

| | | | | | | |
|---|---|---|---|---|---|---|
| CGIC and SGIC score: 1=Very Much Improved, 2=Much Improved, 3=Minimally Improved, 4=No Change, 5= Minimally Worse, 6=Much Worse, 7=Very Much Worse. * P-value is from a t-test comparing the mean score to a score of 4. | | | | | | |

Table 5.1 shows that application of the hydroalcoholic gel effected a statistically significant improvement in descriptive statistics of change rated from photographs within the ITT population.

**Table 5.1**

| Descriptive Statistics of Change from Baseline to Month 6 in Keloid Scar Rated from Photographs ITT Population | |
|---|---|
| | Hydroalcoholic Gel |
| | N=32 |
| n | 31 |
| Mean | 3.0 |
| SD | 0.8 |
| Median | 2.8 |
| Range | (1.6, 4.6) |
| P-value* | <.0001 |

| | |
|---|---|
| Note: Average scores from all assessors are used for analysis. Score: 1=Very Much Improved, 2=Much Improved, 3=Minimally Improved, 4=No Change, 5= Minimally Worse, 6=Much Worse, 7=Very Much Worse * P-value is from a paired t-test comparing the mean to 4. | |

Table 5.2 shows that application of the hydroalcoholic gel effected a statistically significant improvement in descriptive statistics of change rated from photographs within the per protocol population.

**Table 5.2**

| Descriptive Statistics of Change from Baseline to Month 6 in Keloid Scar Rated from Photographs Per Protocol Population | |
|---|---|
| | Hydroalcoholic Gel |
| | N=31 |
| n | 31 |
| Mean | 3.0 |
| SD | 0.8 |
| Median | 2.8 |
| Range | (1.6, 4.6) |
| P-value* | <.0001 |

| | |
|---|---|
| Note: Average scores from all assessors are used for analysis. Score: 1=Very Much Improved, 2=Much Improved, 3=Minimally Improved, 4=No Change, 5= Minimally Worse, 6=Much Worse, 7=Very Much Worse *P-value is from a paired t-test comparing the mean to 4. | |

## Claims

1. A method of treating excessive scars, comprising applying to an excessive scar a pharmaceutical composition that comprises a hydration agent and an anesthetic in the absence of a selective estrogen receptor modulator.

2. The method according to claim 1, wherein said pharmaceutical compositions contains active ingredients consisting solely of one or more hydration agents and one or more anesthetics.

3. The method according to claim 1, wherein said excessive scar is a keloid.

4. The method according to claim 1, wherein said excessive scar is a hypertrophic scar.

5. The method according to claim 1, wherein said hydration agent is an emollient.

6. The method according to claim 5, wherein said emollient is selected from the group consisting of mineral oil, petrolatum, polydecene, isohexadecane, fatty acids and alcohols having from 10 to 30 carbon atoms; pelargonic, lauric, myristic, palmitic, steraric, isostearic, hydroxystearic, oleic, linoleic, ricinoleic, arachidic, behenic, and euricic acids and alcohols; triglyceride esters, castor oil, cocoa butter, safflower oil, sunflower oil, jojoba oil, cottonseed oil, corn oil, olive oil, cod liver oil, almond oil, avocado oil, palm oil, sesame oil, squalene, Kikui oil, soybean oil, acetoglyceride esters, ethoxylated glycerides, ethoxylated glyceryl monostearate, alkyl esters of fatty acids having 10 to 20 carbon atoms, hexyl laurate, isohexyl laurate, isohexyl palmitate, isopropyl palmitate, decyl oleate, isodecyl oleate, hexadecyl stearate, decyl stearate, diisopropyl adipate, diisohexyl adipate, diisopropyl sebacate, laurly lactate, myristyl lactate, acetyl lactate; alkenyl esters of fatty acids having 10 to 20 carbon atoms, oleyl myristate, oleyl stearate, oleyl oleate, fatty acid esters of ethoxylated fatty alcohols, polhydric alcohol esters, ethylene glycol mono and di-fatty acid esters, diethylene glycol mono- and di-fatty acid esters, polyethylene glycol, wax esters, beeswax, spermaceti, myristyl myristate, stearyl stearate, silicone oils, dimethicones, cyclomethicones, and mixtures thereof.

7. The method according to claim 5, wherein said emollient is isopropyl myristate.

8. The method according to claim 1, wherein said hydration agent is a gelling agent.

9. The method according to claim 8, wherein said gelling agent is selected from the group consisting of a carbomer, cellulose derivative, poloxamers, poloxamines, chitosan, dextran, pectin, natural gum, cellulose derivative, ethyl cellulose, hydroxypropyl cellulose, hydroxyethyl cellulose, hydroxypropyl methyl cellulose (HPMC), carboxymethyl cellulose (CMC), and mixtures thereof.

10. The method according to claim 8, wherein said gelling agent is hydroxypropyl cellulose.

11. The method according to claim 1, wherein said hydration agent is a humectant.

12. The method according to claim 11, wherein said humectant is selected from the group consisting of glycerine, propylene glycol, glyceryl triacetate, a polyol, sorbitol, maltitol, a polymeric polyol, polydextrose, quillaia, lactic acid, urea, and mixtures thereof.

13. The method according to claim 1, wherein said anesthetic is selected from the group consisting of alcohols, benzocaine, menthol, butamben, dibucaine, lidocaine, pramoxine, and tetracaine.

14. The method according to claim 1, wherein said alcohol is a lower hydrocarbon chain (C1-C7) alcohol.

15. The method according to claim 14, wherein said alcohol is ethanol or isopropanol.

16. The method according to claim 1, wherein said composition is a hydroalcoholic gel.

17. The method according to claim 16, wherein said hydroalcoholic gel comprises an alcohol, isopropyl myristate, and hydroxypropyl cellulose.

18. The method according to claim 16, wherein said composition comprises :
a) about 0.5% to 2% by weight of isopropyl myristate,
b) about 60% to 75% by weight of absolute alcohol,
c) about 25% to 40% by weight of aqueous vehicle, and
d) about 0.5% to 5% by weight of gelling agent,
wherein the percentage of components are weight to weight of said composition.

19. The method according to claim 18, wherein said isopropyl myristate constitutes about 0.5%, 0.6%, 0.7%, 0.8%, 0.9%, 1.0%, 1.1%, 1.2%, 1.3%, 1.4%, 1.5%, 1.6%, 1.7%, 1.8%, 1.9% or 2.0% by weight of said composition.

20. The method according to claim 18, wherein said alcohol is ethanol or isopropanol, and constitutes about 60% to about 75% by weight of said composition.

21. The method according to claim 18, wherein said gelling agent constitutes about 0.5%, 0.75%, 1.0%, 1.25%, 1.50%, 1.75%, 2.0%, 2.25%, 2.5%, 2.75%, 3.0%, 3.25%, 3.5%, 3.75%, 4.0%, 4.25%, 4.5%, 4.75% or 5.0% by weight of said composition.

22. The method according to claim 18, wherein said gelling agent is a polyacrylic acid, hydroxypropyl cellulose or other cellulose derivative, and constitutes about 0.5% to 5% by weight of said composition.

23. The method according to claim 18, wherein said aqueous vehicle is a phosphate buffered solution, and constitutes about 25% to 40% by weight of the composition.

24. The method according to claim 18, wherein said aqueous vehicle is a phosphate buffered solution, and the pH of said composition is about 4 to about 12, about 6 to about 11, about 8 to about 10, or about 9.

25. The method according to claim 18, wherein said composition further comprises a neutralizing agent selected from the group consisting of sodium hydroxide, ammonium hydroxide, potassium hydroxide, arginine, aminomethylpropanol and tromethamine, which neutralizing agent exists at a neutralizing agent/gelling agent ratio between about 4:1 and 1:1.

26. The method according to claim 1, wherein said composition is applied topically.

27. The method according to claim 1, wherein said composition is applied by injection.

28. A pharmaceutical composition in the form of a hydroalcoholic gel that comprises a hydration agent and an anesthetic in the absence of a selective estrogen receptor modulator or hormone.

29. The pharmaceutical composition according to claim 28, containing active ingredients consisting solely of one or more hydration agents and one or more anesthetics.

30. The pharmaceutical composition according to claim 28, wherein said hydration agent is an emollient.

31. The pharmaceutical composition according to claim 30, wherein said emollient is selected from the group consisting of mineral oil, petrolatum, polydecene, isohexadecane, fatty acids and alcohols having from 10 to 30 carbon atoms; pelargonic, lauric, myristic, palmitic, steraric, isostearic, hydroxystearic, oleic, linoleic, ricinoleic, arachidic, behenic, and euricic acids and alcohols; triglyceride esters, castor oil, cocoa butter, safflower oil, sunflower oil, jojoba oil, cottonseed oil, corn oil, olive oil, cod liver oil, almond oil, avocado oil, palm oil, sesame oil, squalene, Kikui oil, soybean oil, acetoglyceride esters, ethoxylated glycerides, ethoxylated glyceryl monostearate, alkyl esters of fatty acids having 10 to 20 carbon atoms, hexyl laurate, isohexyl laurate, isohexyl palmitate, isopropyl palmitate, decyl oleate, isodecyl oleate, hexadecyl stearate, decyl stearate, diisopropyl adipate, diisohexyl adipate, diisopropyl sebacate, laurly lactate, myristyl lactate, acetyl lactate; alkenyl esters of fatty acids having 10 to 20 carbon atoms, oleyl myristate, oleyl stearate, oleyl oleate, fatty acid esters of ethoxylated fatty alcohols, polhydric alcohol esters, ethylene glycol mono and di-fatty acid esters, diethylene glycol mono- and di-fatty acid esters, polyethylene glycol, wax esters, beeswax, spermaceti, myristyl myristate, stearyl stearate, silicone oils, dimethicones, cyclomethicones, and mixtures thereof.

32. The pharmaceutical composition according to claim 30, wherein said emollient is isopropyl myristate.

33. The pharmaceutical composition according to claim 28, wherein said hydration agent is a gelling agent.

34. The pharmaceutical composition according to claim 33, wherein said gelling agent is selected from the group consisting of a carbomer, cellulose derivative, poloxamers, poloxamines, chitosan, dextran, pectin, natural gum, cellulose derivative, ethyl cellulose, hydroxypropyl cellulose, hydroxyethyl cellulose, hydroxypropyl methyl cellulose (HPMC), carboxymethyl cellulose (CMC), and mixtures thereof.

35. The pharmaceutical composition according to claim 33, wherein said gelling agent is hydroxypropyl cellulose.

36. The pharmaceutical composition according to claim 28, wherein said hydration agent is a humectant.

37. The pharmaceutical composition according to claim 36, wherein said humectant is selected from the group consisting of glycerine, propylene glycol, glyceryl triacetate, a polyol, sorbitol, maltitol, a polymeric polyol, polydextrose, quillaia, lactic acid, urea, and mixtures thereof.

38. The pharmaceutical composition according to claim 28, wherein said anesthetic is selected from the group consisting of alcohols, benzocaine, menthol, butamben, dibucaine, lidocaine, pramoxine, and tetracaine.

39. The pharmaceutical composition according to claim 38, wherein said alcohol is a lower hydrocarbon chain (C1-C4) alcohol.

40. The pharmaceutical composition according to claim 39, wherein said alcohol is ethanol or isopropanol.

41. The pharmaceutical composition according to claim 28, wherein said hydroalcoholic gel comprises an alcohol, isopropyl myristate, and hydroxypropyl cellulose.

42. The pharmaceutical composition according to claim 28, wherein said composition comprises :
a) about 0.5% to 2% by weight of isopropyl myristate,
b) about 60% to 75% by weight of absolute alcohol,
c) about 25% to 40% by weight of aqueous vehicle, and
d) about 0.5% to 5% by weight of gelling agent,
wherein the percentage of components are weight to weight of said composition.

43. The pharmaceutical composition according to claim 42, wherein said isopropyl myristate constitutes about 0.5%, 0.6%, 0.7%, 0.8%, 0.9%, 1.0%, 1.1%, 1.2%, 1.3%, 1.4%, 1.5%, 1.6%, 1.7%, 1.8%, 1.9% or 2.0% by weight of said composition.

44. The pharmaceutical composition according to claim 42, wherein said alcohol is ethanol or isopropanol, and constitutes about 60% to about 75% by weight of said composition.

45. The pharmaceutical composition according to claim 42, wherein said gelling agent constitutes about 0.5%, 0.75%, 1.0%, 1.25%, 1.50%, 1.75%, 2.0%, 2.25%, 2.5%, 2.75%, 3.0%, 3.25%, 3.5%, 3.75%, 4.0%, 4.25%, 4.5%, 4.75% or 5.0% by weight of said composition.

46. The pharmaceutical composition according to claim 42, wherein said gelling agent is a polyacrylic acid, hydroxypropyl cellulose or other cellulose derivative, and constitutes about 0.5% to 5% by weight of said composition.

47. The pharmaceutical composition according to claim 42, wherein said aqueous vehicle is a phosphate buffered solution, and constitutes about 25% to 40% by weight of the composition.

48. The pharmaceutical composition according to claim 42, wherein said aqueous vehicle is a phosphate buffered solution, and the pH of said composition is about 4 to about 12, about 6 to about 11, about 8 to about 10, or about 9.

49. The pharmaceutical composition according to claim 42, wherein said composition further comprises a neutralizing agent selected from the group consisting of sodium hydroxide, ammonium hydroxide, potassium hydroxide, arginine, aminomethylpropanol and tromethamine, which neutralizing agent exists at a neutralizing agent/gelling agent ratio between about 4:1 and 1:1.
